(19) Europäisches Patentamt
European Patent Office
Office européen des brevets

(11) Veröffentlichungsnummer: **0 114 630**
**A2**

# (12) EUROPÄISCHE PATENTANMELDUNG

(21) Anmeldenummer: 84100383.3

(22) Anmeldetag: 16.01.84

(51) Int. Cl.$^3$: **C 12 N 11/16**
C 12 N 11/18, C 12 N 9/04
C 12 N 9/08
//C12P7/58, C12H1/00

(30) Priorität: 21.01.83 DE 3301992

(43) Veröffentlichungstag der Anmeldung:
01.08.84 Patentblatt 84/31

(84) Benannte Vertragsstaaten:
AT BE CH DE FR GB IT LI LU NL SE

(71) Anmelder: BOEHRINGER INGELHEIM KG
D-6507 Ingelheim am Rhein(DE)

(84) Benannte Vertragsstaaten:
BE CH DE FR IT LI LU NL SE AT

(71) Anmelder: Boehringer Ingelheim International G.m.b.H
D-6507 Ingelheim am Rhein(DE)

(84) Benannte Vertragsstaaten:
GB

(72) Erfinder: Hartmeier, Winfried, Prof. Dr.-Ing.
Melatener Strasse 145 a
D-5100 Aachen(DE)

(72) Erfinder: Döppner, Theresia
Hans-Boeckler-Allee 2
D-5100 Aachen(DE)

**(54) Permeabilisiertes Schimmelpilzmycel mit intaktem immobilisiertem Glucoseoxidase-Katalasesystem sowie dessen Herstellung und Anwendung.**

(57) Die Erfindung betrifft permeabilisiertes Schimmelpilzmycel welches immobilisierte Enzyme, z.B. Glucoseoxidase und Katalase sowie gegebenenfalls zusätzlich Enzyme enthält. Die Erfindung betrifft ferner die Herstellung dieses permeabilisierten Schimmelpilzmycels durch Waschen mit wassermischbaren und wasserentziehenden Lösungsmitteln beziehungsweise Ankopplung der Enzyme mittels bi- oder polyfunktioneller Verbindungen sowie die Verwendung des permeabilisierten Schimmelpilzmycels in biotechnischen Reaktionen sowie als Ausgangsmaterial für die Gewinnung löslicher Glucoseoxidase und/oder Katalase.

EP 0 114 630 A2

Die Erfindung betrifft permeabilisiertes Mycel von Schimmelpilzen, das die Enzyme Glucoseoxidase und Katalase in immobilisierter Form enthält. Weiterhin beinhaltet die Erfindung die Herstellung dieses permeabilisierten Schimmelpilzmycels mit immobilisiertem Glucoseoxidase-Katalasesystem sowie dessen Anwendung zur Durchführung biotechnischer Reaktionen, insbesondere der Umsetzung von Glucose zu Gluconolacton bzw. Gluconsäure und der Sauerstoffentfernung aus Bier oder anderen Getränken. Darüberhinaus betrifft die Erfindung auch die Verwendung des permeabilisierten Schimmelpilzmycels, das immobilisierte Glucoseoxidase und Katalase enthält, als Ausgangsmaterial für die Gewinnung besonders reiner löslicher Glucoseoxidase, wie sie besonders für analytische Zwecke benötigt wird. Gegenstand der Erfindung sind weiterhin auch Modifikationen des erfindungsgemäßen permeabilisierten Schimmelpilzmycels, insbesondere Coimmobilisate, die durch Ankopplung weiterer Enzyme an das permeabilisierte Schimmelpilzmycel erhalten werden können, sowie deren Herstellung und Anwendung.

Glucoseoxidase (ß-D-Glucose:Sauerstoff-Oxidoreductase, E.C. 1.1.3.4) katalysiert die Dehydrierungsreaktion von ß-D-Glucose zu D-Glucon-deltalacton. Das gebildete Gluconolacton geht - je nach Reaktionsbedingungen - spontan oder katalysiert durch Lactonase in Gluconsäure über. Der von der Glucose abgespaltene Wasserstoff wird von dem als Cofaktor der Glucoseoxidase fungierenden Flavinadenindinucleotid (FAD) aufgenommen, das dabei in die reduzierte Form ($FADH_2$) überführt wird. Unter Rückbildung von FAD wird der Wasserstoff dann auf Sauerstoff übertragen, so daß Wasserstoffperoxid entsteht. Die von der Glucoseoxidase samt ihrer prosthetischen Gruppe FAD katalysierte Reaktion gehorcht damit der folgenden

Summengleichung:

$$\beta\text{-D-Glucose} + O_2 + H_2O \xrightarrow{\text{Glucoseoxidase}} \text{Gluconsäure} + H_2O_2$$

Für analytische Zwecke - z.B. die Bestimmung von Glucose im Blut oder in Getränken und Lebensmitteln - werden hochgereinigte Glucoseoxidasepräparate eingesetzt. Das gemäß obiger Reaktionsgleichung entstandene Wasserstoffperoxid wird dabei meist mit einer Substanz umgesetzt, die nach der Umsetzung - also im oxidierten Zustand - als chromogener Farbstoff quantitativ im Spektralphotometer erfaßt werden kann.

Normalerweise kommt die das Wasserstoffperoxid spaltende Katalase (Wasserstoffperoxid:Wasserstoffperoxid-Oxidoreductase, E.C. 1.11.1.6) in Bakterien und Pilzen immer gleichzeitig neben der Glucoseoxidase vor. Sie muß aus Präparaten, die dem geschilderten analytischen Zweck dienen sollen, entfernt werden. Weitaus schwieriger als die Entfernung der Katalase ist jedoch die Entfernung von Amyloglucosidase (α-1,4-Glucan-Glucohydrolase, E.C. 3.2.1.3). Amyloglucosidase darf in Glucoseoxidasepräparaten nicht vorhanden sein, wenn diese zur Glucosebestimmung in dextrin- oder stärkehaltigen Substraten (z.B. Getränken oder anderen Lebensmitteln) eingesetzt werden sollen.

Bei Glucoseoxidasepräparaten, die für technische Zwecke (z.B. in der Lebensmittelbe- und -verarbeitung) eingesetzt werden sollen, ist regelmäßig und gewollt soviel Katalase enthalten, daß das durch die Glucoseoxidasereaktion gebildete Wasserstoffperoxid wieder komplett in Wasser und Sauerstoff gespalten wird. Die Katalase-

reaktion gehorcht folgender Summenformel:

$$H_2O_2 \xrightarrow{\text{Katalase}} H_2O + 1/2\ O_2$$

Als Summe von der Glucoseoxidase und der Katalase katalysierten Reaktionen ergibt sich folgende Gesamtreaktion, wenn beide Enzyme in ausreichender Menge im Reaktionsmedium vorhanden sind:

$$\text{Glucose} + 1/2\ O_2 \xrightarrow[\text{Katalase}]{\text{Glucoseoxidase}} \text{Gluconsäure}$$

Eine industrielle Nutzung von Gemischen löslicher Glucoseoxidase und Katalase erfolgt seit langem in der Lebensmittelindustrie zur Entfernung von Glucosespuren oder von Sauerstoff aus Nahrungsmitteln oder Getränken. So wird z.B. lösliche Glucoseoxidase mit Katalase Eiprodukten zugemischt, um Glucosereste daraus zu entfernen, die andernfalls unerwünschte Bräunungsreaktionen (insbesondere bei der Trockeneiherstellung) nach sich ziehen würden. Ein weiteres Anwendungsgebiet ist der Soft-drink-Sektor. Dort dient das dem Getränk zugesetzte Enzymgemisch der Sauerstoffentfernung und damit der Verhinderung von Oxidationsschäden.

Über die genannten Einsatzgebiete hinaus gibt es eine Reihe weiterer Sektoren, bei denen der Einsatz des Glucoseoxidase-Katalasesystems zwar wünschenswert ist, aber nur vereinzelt oder überhaupt nicht praktiziert wird. Haupthinderungsgründe, die einem breiteren Einsatz des Enzymsystems entgegenstehen, sind der hohe Preis und auch die mangelhafte Stabilität des Systems.

Zur Behebung dieser Nachteile wurde insbesondere die Immobilisierung der Glucoseoxidase und der Katalase vorgeschlagen. Durch die Immobilisierung werden die Präparate - zumindest theoretisch - wiederholt oder kontinuierlich einsetzbar, so daß der hohe Enzympreis weniger stark ins Gewicht fällt.

Ein Nachteil der meisten immobilisierten Präparate ist es, daß die Enzymreaktion bei ihnen infolge der Beschränkung der freien Beweglichkeit der Biokatalysatoren und infolge der Einführung zusätzlicher Diffussionsbarrieren für das Substrat bzw. die Substrate behindert ist. Neben diesem Nachteil konnten auch die weiteren üblichen Schwierigkeiten der geringen Enzymdichte und schlechter Substratzuwendung der Enzyme durch das Verfahren nach der DB-PS 2 636 206 überwunden werden.

Die bisher bekannten Immobilisierungsmethoden, z.B. auch das Verfahren gemäß der DB-PS Nr. 2 636 206, gehen allgemein von löslicher Glucoseoxidase und löslicher Katalase aus, die dann an feste (unlösliche) Trägerstoffe gebunden werden. Diese Immobilisierungsprozedur gemäß dem Stand der Technik hat den Nachteil, daß nur relativ wenig der vom Schimmelpilzmycel gebildeten Glucoseoxidase- und Katalaseaktivität in biokatalytisch aktiver Form in die immobilisierte Form überführt wird. Ein Großteil der erwünschten Enzymaktivität geht bereits vor der eigentlichen Immobilisierung bei der Aufarbeitung des Mycels zu löslicher Glucoseoxidase und Katalase verloren; weitere Aktivitätsverluste treten dann bei der Ankopplung der löslichen Enzyme an feste Trägerstoffe auf. Nachfolgende Tabelle 1 verdeutlicht dies mit den Daten von Beispiel 1 der DB-PS 2 636 206.

Tabelle 1

| | |
|---|---|
| Aktivität des Mycels ex Fermentation ........ | 2000 Su/g |
| Aktivitätsverlust bei Aufarbeitung zu löslichem Enzym ............................ | 60 % |
| Verbleibende Aktivität vom Ausgangsmycel .... | 800 Su/g |
| Aktivitätsverlust bei Immobilisierung ....... | 82 % |
| Verbleibende Aktivität vom Ausgangsmycel im Immobilisat ............................ | 140 Su/g |

Ein weiterer gravierender Nachteil der bisher bekannten nativen und immobilisierten Glucoseoxidase-Katalasepräparate ist ihre Instabilität gegenüber Wasserstoffperoxid. Eine höhere Stabilität wäre insbesondere bei der technischen Glucoseoxidation zu Gluconsäure wünschenswert. Bei diesem Prozeß muß Sauerstoff in großer Menge zur Aufrechterhaltung des Reaktionsablaufes in die Reaktionslösung eingebracht werden. Eine besonders schnelle Einbringung von Sauerstoff ist durch Zugabe von Wasserstoffperoxid möglich, wie es u.a. von G. Richter und H. Heinecker (Stärke 31, 418-422) 1979)) vorgeschlagen wurde. Das Peroxid wird durch die im Enzymgemisch vorhandene Katalase in Sauerstoff und Wasser gespalten und kann somit zur Sauerstoffversorgung eingesetzt werden. Leider ist das Enzymsystem aber nach bisherigen Erkenntnissen unter diesen Bedingungen nicht hinreichend stabil.

Eine gewisse - aber nach wie vor unzureichende - Verbesserung der Stabilität immobilisierter Glucoseoxidase-Katalasepräparate konnte durch Verwendung von die Peroxidspaltung fördernden anorganischen Trägermaterialien - wie z.B. Manganoxid oder Rutheniumverbindungen - erreicht werden. Ein derartiger Vorschlag wurde von R. Carter, J.E. Prenosil und J.R. Bourne

(Preprints Part 1, S. 107-108, First Europ. Congress Biotechnology, Interlaken, 1978) unterbreitet. Die Halbwertzeit, das ist die bis zur Aktivitätsabnahme auf die Hälfte des ursprünglichen Wertes erforderliche Zeit, ist jedoch auch bei diesen Präparaten unter den vorgeschlagenen Reaktionsbedingungen auf wenige Tage beschränkt. Ein weiterer beträchtlicher Nachteil der Verwendung der genannten anorganischen Katalysatoren ist auch deren lebensmittelrechtliche Bedenklichkeit.

Für die großtechnische Produktion von Gluconsäure aus Glucose oder aus Invertzucker findet derzeit weder das lösliche noch das immobilisierte Glucoseoxidase-Katalasesystem Anwendung, obwohl eine Reihe derartiger Vorschläge aus der Fach- und Patentliteratur bekannt sind. US-PS 3 050 444 beschreibt ein Verfahren, bei dem Glucose, die in Invertzucker (= Glucose + Fructose) vorkommt, zu Gluconsäure oxidiert wird. Die gebildete Gluconsäure läßt sich z.B. durch Fällung als Calciumgluconat wesentlich leichter von der Fructose abtrennen als die ursprünglich vorhandene Glucose. Das Verfahren wurde in den 60er Jahren in größerem Maßstab zur Gewinnung von Fructose und Gluconat eingesetzt. Infolge des relativ hohen Preises für das Enzymgemisch ist das Verfahren heute nicht mehr konkurrenzfähig.

Um den hohen Enzympreis zu kompensieren wird in DE-AS 20 63 732 vorgeschlagen, die teure Glucoseoxidase durch Elektrodialyse aus der Reaktionslösung wiederzugewinnen und dann wiederholt in löslicher Form einzusetzen. Laut dieser DE-AS konnten mit 0,688 g handelsüblicher löslicher Glucoseoxidase 830 g Natriumgluconat hergestellt werden, d.h. mit 1 g löslicher Glucoseoxidase können nach diesem Verfahren 1206 g Natriumgluconat erzielt werden. Das Verhältnis von Gluconausbeute pro eingesetzter Glucoseoxidase konnte durch unser eigenes Verfahren nach DE-OS 29 11 192 auf rund 10 000 g Gluconat aus einem Gramm löslicher Ausgangsglucosidase gesteigert werden. Die dabei verwendete Glucoseoxidase war einer Immobilisierung gemäß der schon erwähnten DE-PS 2 636 206 unterworfen worden. Ein beträchtlicher Nachteil des Verfahrens nach DE-AS 20 03 732 ist es auch, daß die Elektrodialyse eine komplizierte Apparatur erfordert, die sich im rauhen Praxisbetrieb nur schwer beherrschen läßt; die erforderlichen Mehrmembran-Dialyseapparate sind anfällig gegen Beschädigung und Störungen.

Wegen der schon erwähnten Nachteile isolierter löslicher und trägergebundener Glucoseoxidase-Katalasemischungen wird Gluconat heute immer noch bevorzugt durch direkte Fermentation mit Bakterien (z.B. Pseudomonas ovalis, Gluconobacter oxydans) oder Schimmelpilzen (z.B. Aspergillus niger) gewonnen. Diese Fermentation mit intakten Zellen oder Mycelien hat den Nachteil, daß Teile des Substrates (Glucose) in zelleigene Substanzen und zu anderen unerwünschten Stoffwechselprodukten umgewandelt werden.

Aufgabe der vorliegenden Erfindung war es, ein kostengünstiges Präparat zu schaffen, das Glucoseoxidase und Katalase in immobilisierter Form enthält. Die Aktivitätsverluste, die bei der bisherigen Praxis der Isolierung und Reinigung des Glucoseoxidase-Katalysesystems aus Schimmelpilzmycel und der anschließenden Bindung der Enzyme zu einem immobilisierten System üblich waren, sollten bei dem neuentwickelten immobilisierten Glucoseoxidase-Katalasesystem nicht mehr gegeben sein. Weitere, in besonderen Ausführungsformen der Erfindung angestrebte Vorteile gegenüber dem bisherigen Stand der Technik waren es insbesondere, eine höhere Stabilität gegenüber

Wasserstoffperoxid und eine bessere Wirksamkeit bei der Sauerstoffentfernung aus Bier oder anderen Getränken zu erzielen. Weiterhin war es Aufgabe der Erfindung, eine Methode zur einfachen Abtrennung von störenden Nebenaktivitäten (wie z.B. Amyloglucosidase) zu finden. Schließlich sollten Verfahren zur Anwendung des Glucoseoxidase-Katalasesystems in der Lebensmittel- und Biotechnologie ausgearbeitet werden, die apparate- und verfahrenstechnisch einfach und lebensmittelrechtlich möglichst unbedenklich waren.

Es wurde gefunden, daß sehr kostengünstig und praktisch ohne Aktivitätsverlust aus glucoseoxidase- und katalasehaltigem Schimmelpilzmycel ein Präparat mit Glucoseoxidase und Katalase in immobilisierter Form erhalten werden kann, wenn das Schimmelpilzmycel durch geeignete Behandlung permeabilisiert wird. Für die Permeabilisierung kommt bevorzugt eine Waschung des Mycels mit hierzu geeigneten wassermischbaren und wasserentziehenden Lösungsmitteln, wie z.B. iso-Propanol, Methanol, Äthanol, Aceton, Äther u.a. in Frage. Weiterhin kann eine Permeabilisierung auch durch schonende Trocknung, insbesondere Vakuumtrocknung oder Sprühtrocknung und anschließende Waschung mit Lösungsmitteln oder Schockbehandlung mit kaltem Wasser erzielt werden.

Vor Anwendung bzw. vor Inverkehrbringen des permeabilisierten Mycels wird dieses üblicherweise zur Entfernung löslicher Mycelinhaltsstoffe mit Wasser gewaschen. Grundsätzlich ist es aber auch möglich, das permeabilisierte Mycel mit noch vorhandenen löslichen Bestandteilen für biotechnische Reaktionen einzusetzen. In bevorzugter Ausführungsform ist das permeabilisierte Mycel gewaschen und damit überwiegend

oder ganz von löslichen Komponenten befreit. Weiterhin ist es Sache des Anwendungsgebietes und der gegebenen Erfordernisse und der Wünsche des Anwenders, ob das permeabilisierte Mycel in wäßriger Suspension mit oder ohne Konservierungsstoff(e) oder in schonend getrockneter (z.B. vakuumgetrockneter, sprühgetrockneter) Form bis zur Verwendung kühl (z.B. 4°C) aufbewahrt wird.

Ein beträchtlicher Vorteil des permeabilisierten Schimmelpilzmycels gemäß Erfindung ist es, daß es annähernd die gleiche Glucoseoxidase- und Katalaseaktivität aufweist wie das lebende Ausgangsmycel. Aktivitätsverluste, wie sie sonst bei der Isolierung, Reinigung und Immobilisierung von Glucoseoxidase und Katalase üblich sind, treten nur gering auf. Das frische (feuchte) oder schonend getrocknete Mycel wird mit geringem Aktivitätsverlust von ca.25 % in die permeabilisierte, die Enzyme Glucoseoxidase und Katalase immobilisiert enthaltende Form überführt. Es verbleiben demnach im neuen erfindungsgemäßen Präparat rund 75 % der im Ausgangsmycel enthaltenen Glucoseoxidaseaktivität, während in immobilisierten Präparaten gemäß dem Stand der Technik (siehe Tabelle 1) nur etwa 7 % der Aktivität des Ausgangsmycels aktiv erhalten bleiben.

Gegenüber nativem Ausgangsmycel hat das permeabilisierte Mycel u.a. die Vorteile, daß es nicht mehr lebensfähig ist und im wesentlichen nur die Enzyme Glucoseoxidase und Katalase in immobilisierter, d.h. unlöslicher aber biokatalytisch wirksamer Form enthält. Unerwünschte Nebenreaktionen sind damit ausgeschaltet. Insbesondere die stark störenden Begleitaktivitäten z.B. von Amyloglucosidase, ß-Glucanasen, Proteasen u.a. liegen im permeabilisierten Mycel nicht in immobilisierter Form vor. Da das permeabilisierte Mycel üblicherweise vor

Einsatz gewaschen wird bzw. in gewaschener Form in den Handel gelangt, kommen diese Nebenaktivitäten bei Verwendung des Präparates nicht mehr störend zum Tragen. Gegenüber nativem Ausgangsmycel hat das permeabilisierte Präparat keine die Diffusion hemmenden Membranschranken, weil diese durch die Permeabilisierung aufgehoben sind.

Grundsätzlich kann das permeabilisierte Präparat gemäß der Erfindung aus jedem Schimmelpilzmycel hergestellt werden, das Glucoseoxidase und Katalase enthält. In besonderem Maße eignet sich natürlich Mycel, dessen Glucoseoxidasegehalt besonders hoch, z.B. über 2000 Sarett-Einheiten pro Gramm Mycel-Trockensubstanz, liegt. Es ist Sache des Fachmannes hierzu einen geeigneten Pilzstamm, z.B. aus Stammsammlungen oder direkt aus der Natur zu isolieren. Bevorzugt werden Schimmelpilze zum Einsatz gelangen, wie sie bisher schon zur technischen Gewinnung von Glucoseoxidase und Katalase eingesetzt werden. Diese Schimmelpilze gehören meist den Gattungen Aspergillus und Penicillium an. Besonders Stämme der Art Aspergillus niger und dessen Varietäten werden gerne zur Glucoseoxidasegewinnung verwendet und sind somit auch in besonderem Maße geeignet zur Herstellung des erfindungsgemäßen Präparates.

Zur Anzucht der Schimmelpilze sind solche Kulturbedingungen einzustellen, die zu möglichst hoher Glucoseoxidase- und Katalasebildung bei den Pilzen führen. Hierzu können Bedingungen angewandt werden, wie sie z.B. von K.Z. Zetelaki (Biotechnol. Bioengng. 10, 45-59 (1968) und Biotechnol. Bioengng. 12, 379-397 (1970) und von W. Franke (Arch.Mikrobiol. 49, 64-80 (1964)) angegeben werden. Meist wird bei der Fermentation zur Glucoseoxidaseproduktion im Submersverfahren

mit Glucoseüberschuß, starker Belüfung, Stickstoff- und Phosphatmangel gearbeitet. Grundätzlich liegt es aber beim Fachmann, die für die Glucoseoxidase- und Katalasebildung optimalen Kulturbedingungen zu ermitteln und anzuwenden.

Die Glucoseoxidase- und Katalaseaktivität des erfindungsgemäßen Präparats kann grundsätzlich beliebig hoch sein. Besonders günstig ist eine möglichst hohe Aktivität beider Enzyme. Technisch geeignet sind insbesondere Präparate, die eine Mindestaktivität von 1000 Sarett-Einheiten Glucoseoxidase und 250 Baker-Einheiten Katalase pro Gramm Trockensubstanz aufweisen.

In einer bevorzugten Ausführungsform des erfindungsgemäßen Präparates liegt dieses in modifizierter Form (d.h. gebunden an weitere Materialien oder Wirkstoffe) vor. Als modifizierte Form ist insbesondere das Coimmobilisat des permeabilisierten - Katalase und Glucosioxidase enthaltenden - Mycels mit weiteren Enzymen (z.B. Amyloglucosidase oder zusätzlicher Katalase) zu verstehen.

Zur Herstellung eines Coimmobilisats gemäß der Erfindung wird das mehr oder weniger entwässerte permeabilisierte Mycel in eine wäßrige Lösung desjenigen Enzyms eingebracht, mit dem coimmobilisiert werden soll. Die Wassermenge der Enzymlösung ist dabei so zu bemessen, daß das Mycel bei seiner Rehydratisierung (Wiederbefeuchtung) das Wasser der Enzymlösung zum größten Teil aufsaugt. Bei dem Rehydratisierungsvorgang werden die Enzyme an die Mycelwände herangesogen, an die sie im nachfolgenden Bindungsprozeß angekoppelt und weiterhin untereinander quervernetzt werden.

Um die Enzymmoleküle nach der Wiederbefeuchtung des permeabilisierten Mycels auf der Mycelwand bzw. in deren unmittelbarer Nähe liegend zu erhalten, ist der Zusatz einer enzymfällenden Substanz gleichzeitig oder vor Zugabe eines Vernetzungsmittels zweckmäßig. Dieser Zusatz bewirkt auch, daß die Mycelfäden nicht unerwünschtermaßen agglomerieren. Als enzymfällende Mittel können z.B. organische Lösungsmittel (i-Propanol, Äthanol, Aceton u.a.) oder Tannin oder andere in solcher Menge eingesetzt werden, daß ihre enzymfällende Wirkung zum Tragen kommt. Bei Verwendung von iso-Propanol müssen meist 20 bis 70 % dieses Lösungsmittels im Reaktionsansatz vorhanden sein. Bei Verwendung von Tannin genügen meist 0,2 bis 5,0 % im Reaktionsansatz.

Als Vernetzungs- und Bindemittel bei der Coimmobilisierung können allgemein bekannte bi- oder polyfunktionelle Verbindungen - wie z.B. Glutardialdehyd, Hexamethylendiamin oder Hexamethylentetramin - einzeln oder in Kombination eingesetzt werden. Bevorzugt wird Glutardialdehyd in einer Konzentration von 0,1 bis 10 % im Reaktionsgemisch verwendet. Die Vernetzung wird wenige Minuten bis zu mehrere Tage lang, bevorzugt 1 bis 3 Stunden, bei vorzugsweise 20 bis 30°C durchgeführt. Dabei ist es günstig, den Reaktionsansatz z.B. durch Rühren oder Schütteln etwas zu bewegen. Nach Beendigung der Vernetzung wird das so hergestellte Coimmobilisat in der Regel gewaschen und im feuchten oder schonend getrockneten Zustand seiner weiteren Verwendung zugeführt.

Eine besonders vorteilhafte Ausführungsform der Coimmobilisierung sieht vor, daß sie unter Zugabe einer quaternären Ammoniumverbindung (z.B. des Handels-

produktes "Absonal®") vorgenommen wird. Es wurde nämlich gefunden, daß der Aktivitätserhalt von Glucoseoxidase bei der Coimmobilisierungsprozedur von 40 auf 70 % gesteigert wird, wenn eine quaternäre Ammoniumverbindung verwendet wird. Dieses Ergebnis ist überraschend, weil allgemein angenommen wird, daß quaternäre Ammoniumverbindungen die Enzymaktivitäten hemmen und enzyminaktivierend wirken. Die Zusatzmenge von quaternären Ammoniumverbindungen liegt vorzugsweise zwischen 0,02 und 0,2 % der Enzymlösung, die zur Coimmobilisierung eingesetzt wird.

Ein besonders vorteilhaftes Coimmobilisat besteht aus permeablisiertem Schimmelpilzmycel mit immobilisiertem intracellulärem Glucoseoxidase-Katalasesystem und angekoppelter zusätzlicher Katalase. Ein derartiges Coimmobilisat hat bei Anwendung zur enzymatischen Glucoseoxidation eine erheblich verbesserte Stabilität im Vergleich zum normalen permeabilisierten Schimmelpilzmycel sowie erst recht im Vergleich zu immobilisierten Glucoseoxidase-Katalasepräparaten gemäß dem Stand der Technik. Das Coimmobilisat weist vor allem gegenüber Wasserstoffperoxid eine verbesserte Stabilität auf, so daß die Verwendung von Wasserstoffperoxid zur Sauerstoff-Versorgung der Reaktionsansätze ermöglicht wird.

Ein weiteres besonders vorteilhaftes Coimmobilisat entsteht, wenn Amyloglucosidase an das permeabilisierte Schimmelpilzmycel angekoppelt wird. Dieses Coimmobilisat ist z.B. in hervorragender Weise geeignet, Sauerstoff aus Getränken - insbesondere Bier - zu entfernen. Die an das Mycel angekoppelte Amyloglucosidase setzt aus den Dextrinen des Bieres Glucose frei, die als Reaktions-

partner der Glucoseoxidasereaktion die Sauerstoff-Entfernung aus dem Getränk beträchtlich beschleunigt.

Das permeabilisierte Schimmelpilzmycel und seine Modifikationen können zur Durchführung biotechnischer Reaktionen eingesetzt werden. Besonders bevorzugte Einsatzgebiete sind die Glucoseoxidation zu Gluconolacton und Gluconsäure sowie die Sauerstoffentfernung aus Getränken, wie z.B. Bier.

Das permeabilisierte Schimmelpilzmycel gemäß der Erfindung kann auch als Ausgangsmaterial zur Gewinnung löslicher Glucoseoxidase und/oder Katalase Verwendung finden. Der Vorteil des Einsatzes von permeabilisiertem Schimmelpilzmycel für diesen Zweck besteht darin, daß in ihm praktisch nur Glucoseoxidase und Katalase in gebundener Form vorliegen. Begleitenzyme können demnach leicht herausgewaschen werden bzw. sie sind herausgewaschen. Durch anschließende enzymatische, mechanische oder sonstige Lösung der immobilisierten Glucoseoxidase und/oder Katalase können diese Enzyme z.B. völlig frei von Amyloglucosidase gewonnen werden. Für die Lösungsprozedur nach enzymatischer Methode kommt z.B. das Verfahren, das in der DB-PS 2 636 636 für die Invertasefreisetzung beschrieben ist, infrage.

## Beispiel 1

**Mycelherstellung und Permeabilisierung mit iso-Propanol**

20 l Kulturmedium wurden im Fermenter mit Konidien des Aspergillus niger Stammes DSM 823 aus der Deutschen Sammlung für Mikroorganismen (Göttingen) beimpft. Der Fermenter wurde mit 40 Ml/min belüftet, mit 500 UpM gerührt, auf 28°C temperiert und der pH-Wert durch Titration mit 10 N NaOH auf 4,5 gehalten. Nach 30 bis 40 Stunden wurde das Mycel durch Druckfiltration geerntet. Das abfiltrierte Mycel wurde mit Wasser gewaschen und trockengesaugt. Die anschließende Permeabilisierung erfolgte durch Aufnahme des Mycels in eiskaltem (5°C) iso-Propanol. Nach guter Durchmischung wurde es wiederum abgesaugt, im Vakuumtrockenschrank bei 35°C getrocknet und mit einem Handrührgerät pulverisiert. Die Aufbewahrung erfolgte im getrockneten Zustand bei 5°C. Zur Bestimmung der Glucoseoxidaseaktivität wurde das getrocknete, permeabilisierte Mycel in Citrat (0,1 m) Phosphat (0,2 m)-Puffer pH 6,0 aufgenommen, homogenisiert und zur Glucoseoxidaseaktivitätsbestimmung eingesetzt. Es konnten für das permeabilisierte Mycel 2000 Sarett-einheiten pro Gramm Trockensubstanz Glucoseoxidase-aktivität bestimmt werden.

Die Zusammensetzung des Kulturmediums ist wie folgt:

| | |
|---|---|
| 300 g | Saccarose/l |
| 3,5 g | $NaNO_3$/l |
| 1,25 g | $MgSO_4 \cdot 7H_2O$/l |
| 1,25 g | KCl/l |
| 0,025 g | $FeSO_4 \cdot 7H_2O$/l |
| 2,5 g | $K_2H\ PO_4$/l |
| pH 7,3 | (NaOH) |

Beispiel 2

Permeabilisierung durch Sprühtrocknung und Behandlung mit kaltem Wasser

Die Fermentation des Mycels wurde wie in Beispiel 1 durchgeführt. Die Aufarbeitung des Fermenterinhaltes erfolgte durch Filtration und anschließende Waschung des Mycels. Das so behandelte Mycel wurde sprühgetrocknet, wobei die Luft-Eingangstemperatur bei 140°C und die Luft-Ausgangstemperatur bei 70°C lag und anschließend in eiskaltem (5°C) Wasser resuspendiert. Nach gründlicher Mischung wurde das Mycel abgetrennt und im Vakuumtrockenschrank bei 35°C getrocknet. Bis zur weiteren Verwendung erfolgte eine Aufbewahrung im Kühlschrank bei 5°C. Bei diesem Permeabilisierungsprozeß wurde eine Glucoseoxidaseaktivität des permeabilisierten, getrockneten Mycels von 1780 Saretteinheiten pro Gramm Trockensubstanz bestimmt.

Beispiel 3

Coimmobilisierung mit Katalase

0,4 g Albumin wurden in einem 300 ml Erlenmeyerkolben mit 30 ml destilliertem Wasser gelöst und mit 20 ml Katalase-Lösung versetzt. In diese Lösung wurden 5,0 g getrocknetes, permeabilisiertes Mycel eingerührt. 80 ml Isopropanol wurden zunächst in einem Becherglas mit 8,0 ml 25 %igem Glutardialdehyd gemischt und dann dem Albumin-Mycel-Katalase-Gemisch zugesetzt. Der Ansatz wurde 2 h bei 25°C im Schüttelwasserbad inkubiert und anschließend filtriert. Der Rückstand wurde zweimal mit je 200 ml eiskaltem dest. Wasser gewaschen, in 200 ml Citrat (0,1m)-Phosphat (0,2 m)-Puffer pH 5,0 gegeben und 24 h bei 5°C stehengelassen. Daran anschließend wurde das Coimmobilisat

filtriert, mehrmals mit eiskaltem dest. Wasser gewaschen, auf 350 ml Citrat (0,1 m)-Phosphat(0,2 m)-Puffer pH 5,0 + 0,2 % Absonal® aufgefüllt und bei 5°C aufbewahrt. Die Glucoseoxidaseaktivität des Coimmobilisats konnte mit 800 Saretteinheiten pro Gramm Mycel-Trockensubstanz bestimmt werden.

Beispiel 4

Coimmobilisierung mit Katalase unter Absonal®-Zusatz

0,4 g Albumin wurden in einem 300 ml Erlenmeyerkolben mit 30 ml dest. Wasser gelöst und mit 20 ml Katalase-Lösung versetzt, der zuvor 0,2 % Absonal® zugesetzt wurden. Die Katalase-Lösung hatte eine Aktivität von 2600 Bakereinheiten pro Milliliter Lösung und von 200 Saretteinheiten pro Milliliter Lösung. In diese Lösung wurden 5,0 g getrocknetes, permeabilisiertes Mycel eingerührt. 80 ml iso-Propanol wurden zunächst in einem Becherglas mit 8,0 ml 25 %igem Glutardialdehyd gemischt und dann dem Albumin-Mycel-Katalase-Gemisch zugesetzt. Der Ansatz wurde 2 h bei 25°C im Schüttelwasserbad inkubiert und anschließend filtriert. Der Rückstand wurde zweimal mit je 200 ml eiskaltem dest. Wasser gewaschen, in 200 ml Citrat(0,1 m)-Phosphat (0,2 m)-Puffer pH 5,0 gegeben und 24 h bei 5°C stehengelassen. Daran anschließend wurde das Coimmobilisat filtriert, mehrmals mit eiskaltem dest. Wasser gewaschen, auf 350 ml Citrat(o,1 m)-Phosphat (0,2 m)-Puffer pH 5,0 + 0,2 % Absonal® aufgefüllt und bei 5°C aufbewahrt. Als Aktivitätswerte konnten bezüglich Glucoseoxidase 2000 Saretteinheiten pro Gramm Mycel-Trockensubstanz und bezüglich Katalase 7600 Bakereinheiten pro Gramm Mycel-Trockensubstanz ermittelt werden.

Die Michaeliskonstante des Coimmobilisats wurde betreffs Glukose mit $K_m$ = 10,6 mMol/l bestimmt und lag damit um gut die Hälfte niedriger als die Michaeliskonstante gemäß dem nach DB-PS 2 636 206 hergestellten Immobilisationspräparat.

Beispiel 5

Coimmobilisation mit Amyloglucosidase unter Absonal®-zusatz

0,4 g Albumin wurden in 30 ml dest. Wasser gelöst und mit 20 ml Amyloglucosidase-Lösung, die 0,2 % Absonal® enthielt, versetzt. Dazu wurden 10 g eines handelsüblichen Trockenpräparates der Amyloglucosidase mit 1070 Glucoamylase-Einheiten pro Gramm in 100 ml dest. Wasser gelöst, so daß eine Enzymlösung von 107 Amyloglucosidase-Einheiten pro Milliliter eingesetzt werden konnte. In die Albumin-Amyloglucosidase-Lösung wurden 5,0 g getrocknetes, permeabilisiertes Mycel eingerührt. 80 ml iso-Propanol wurden zunächst in einem Becherglas mit 8,0 ml 25 %igem Glutardialdehyd gemischt und dann dem Albumin-Mycel-Amyloglucosidase-Gemisch zugesetzt. Der Ansatz wurde 2 h bei 25°C im Schüttelwasserbad inkubiert und anschließend filtriert. Der Rückstand wurde zweimal mit je 200 ml eiskaltem dest. Wasser gewaschen, in 200 ml Citrat(0,1m)-Phosphat (0,2 m)-Puffer pH 5,0 gegeben und 24 h bei 5°C stehengelassen. Daran anschließend wurde das Coimmobilisat filtriert, mehrmals mit eiskaltem dest. Wasser gewaschen, auf 350 ml Citrat (0,1 m)-Phosphat(0,2 m)-Puffer pH 5,0 + 0,2 % Absonal® aufgefüllt und bei 5°C aufbewahrt. Die Aktivitätsbestimmung erbrachte eine Glucoseoxidaseaktivität von 1015 Saretteinheiten pro Gramm Mycel-Trockensubstanz und bezüglich Amyloglucosidase eine Aktivität von 170 Amyloglucosidaseeinheiten pro Gramm Mycel-Trockensubstanz.

Beispiel 6

Wiederholter Einsatz von Coimmoblisat gemäß Beispiel 4 zur Glucoseoxidation

Das gemäß Beispiel 4 hergestellte Coimmobilisat wurde zur Glucoseoxidation im Rührreaktor eingesetzt. Der Reaktor wurde mit 0,2 l 10 %iger Glucoselösung pH 5,5 befüllt. Der pH-Wert wurde während der Reaktionszeit durch Titration mit 2 n NaOH konstant gehalten. Ein Temperiermantel gewährleistete eine Temperatur von 5°C. Die Sauerstoffkonzentration wurde mittels einer Sauerstoffelektrode gemessen und über einen Regler mit Titrationsvorrichtung durch Zugabe von 30 %igem Wasserstoffperoxid auf 18-20 mg $O_2$/l eingestellt. Der Verbrauch an Natronlauge wurde über der Zeit registriert. Er entsprach der gebildeten Gluconsäure und gab den Reaktionsverlauf wieder. Bei Erreichen eines Konversionsgrades von ca. 80 % wurde die Umsetzung abgebrochen, das Enzympräparat abfiltriert, gründlich gewaschen und nach Bestimmung der Glucoseoxidase- und Katalaseaktivitätswerte nochmal in gleicher Weise eingesetzt. Bei Einsatz von 1 Gramm Coimmobilisat, das eine Aktivität von 2000 Su/g bezüglich Glucoseoxidase und von 7600 Bu/g bezüglich Katalase aufwies, wurden nach einer Umsetzung 10 % Verlust bezüglich Glucoseoxidase und 20 % Verlust bezüglich Katalase verzeichnet. Bei der zweiten Umsetzung traten nochmals Verluste von 9 % bezüglich der Glucoseoxidase und 22 % bezüglich der Katalase auf.

Beispiel 7

Einsatz von Coimmobilisat gemäß Beispiel 5 zur Sauerstoffentfernung aus Bier

Das gemäß Beispiel 5 hergestellte Coimmobilisat wurde filtriert, mehrmals mit Wasser gewaschen und in Wasser

aufgenommen. Anschließend wurde es mit dem Ultra-Turrax homogenisiert, mit Perlite B im Verhältnis 1 : 4 gemischt und mit 4 bar Druck in einem Rahmenreaktor angeschwemmt. Das sauerstoffbelastete Bier wurde mit 2 bar Arbeitsdruck durch den Reaktor gedrückt. Dabei wurde mit verschiedenen Durchflußraten gearbeitet. Die Sauerstoffwerte vor und nach der Behandlung wurden mittels eines 2-Kanalschreibers aufgezeichnet. Die ermittelten Durchflußraten sind in Tabelle 2 wiedergegeben.

Tabelle 2

| stündliche Durchflußrate absolut l/h | stündliche Durchflußrate spez. l/h kg | $O_2$-Gehalt vor Reaktor mg/l | $O_2$-Gehalt nach Reaktor mg/l |
|---|---|---|---|
| 12,30 | 3892 | 0,84 | 0,04 |
| 46,80 | 14715 | 0,84 | 0,10 |
| 54,00 | 17088 | 0,80 | 0,13 |
| 10,05 | 3180 | 2,36 | 0,09 |
| 39,60 | 12531 | 2,30 | 0,22 |
| 54,00 | 17088 | 2,40 | 0,35 |
| 60,00 | 18987 | 2,36 | 0,37 |
| 5,40 | 1708 | 4,60 | 0,05 |
| 10,00 | 3164 | 4,50 | 0,13 |
| 17,55 | 5553 | 4,40 | 0,27 |
| 10,00 | 8354 | 4,50 | 0,41 |

Enzymaktivitätsbestimmungen

Die Bestimmung der Enzymaktivitäten erfolgt nach den nachfolgend angegebenen Methoden. Im Falle von fixierten Enzymen wurden die Inkubationsansätze gerührt oder geschüttelt, auch wenn das in der Originalvorschrift nicht angegeben war.

Katalaseaktivität:
nach First Suppl.Food Chem. Codex, 2nd. Ed., S. 67-68, Verlag National Academy of Sciences, Washington, 1974.

Glucoseoxidaseaktivität:
nach First Suppl. Food Chem. Codex, 2nd Ed., S. 78-79, Verlag National Academy of Sciences, Washington, 1974.

Amyloglucoseaktivität:
nach H.J. P i e p e r, Mikrobielle Amylasen bei der Alkoholgewinnung. S. 48-49, Verlag Ulmer, Stuttgart.

Patentansprüche

1. Permeabilisiertes Schimmelpilzmycel dadurch gekennzeichnet, daß es immobilisierte Enzyme enthält.

2. Permeabilisiertes Schimmelpilzmycel nach Anspruch 1, dadurch gekennzeichnet, daß es die Enzyme Glucoseoxidase und Katalase in immobilisierter Form enthält.

3. Permeabilisiertes Schimmelpilzmycel nach Anspruch 1, dadurch gekennzeichnet, daß es im Wesentlichen keine anderen Enzyme außer Glucoseoxidase und Katalase in immobilisierter Form enthält.

4. Permeabilisiertes Schimmelpilzmycel nach Anspruch 1, dadurch gekennzeichnet, daß die Enzyme durch Auswaschen mit wasserentziehenden Lösungsmitteln immobilisiert worden sind.

5. Permeabilisiertes Schimmelpilzmycel nach Anspruch 1, dadurch gekennzeichnet, daß die Enzyme durch kovalente Vernetzung immobilisiert worden sind.

6. Permeabilisiertes Schimmelpilzmycel nach einem der Ansprüche 1 bis 5, dadurch gekennzeichnet, daß es eine Glucoseoxidaseaktivität von mindestens 1000 Saretteinheiten pro Gramm Trockensubstanz und eine Katalaseaktivität von mindestens 250 Bakereinheiten pro Gramm Trockenstubstanz aufweist.

7. Permeabilisiertes Schimmelpilzmycel nach einem der Ansprüche 1 bis 6, dadurch gekennzeichnet, daß es von Pilzen der Art Aspergillus niger oder Varietäten dieser Art stammt.

8. Permeabilisiertes Schimmelpilzmycel nach einem der Ansprüche 1 bis 7, dadurch gekennzeichnet, daß es zusätzlich modifiziert ist.

9. Permeabilisiertes und zusätzlich modifiziertes Schimmelpilzmycel nach Anspruch 8, dadurch gekennzeichnet, daß es zusätzlich animmobilisierte(s) Enzym(e) enthält.

10. Permeabilisiertes und zusätzlich modifiziertes Schimmelpilzmycel nach Anspruch 9, dadurch gekennzeichnet, daß es Katalase als zusätzlich animmobilisiertes Enzym enthält.

11. Permeabilisiertes und zusätzlich modifiziertes Schimmelpilzmycel nach Anspruch 9, dadurch gekennzeichnet, daß es Amyloglucosidase als zusätzlich animmobilisiertes Enzym enthält.

12. Verfahren zur Herstellung von permeabilisiertem Schimmelpilzmycel nach einem der Ansprüche 1 bis 11, dadurch gekennzeichnet, daß glucoseoxidase- und katalasehaltiges Schimmelpilzmycel mit einem oder mehreren Lösungsmitteln gewaschen und gewünschtenfalls anschließend die Enzyme in wäßrigem Medium mittels bi- oder polyfunktioneller Verbindungen angekoppelt werden.

13. Verfahren zur Herstellung von permeabilisiertem Schimmelpilzmycel nach einem der Ansprüche 1 bis 11, dadurch gekennzeichnet, daß glucoseoxidase- und katalasehaltiges Schimmelpilzmycel zunächst schonend getrocknet, dann mit kaltem Wasser oder

kalter wäßriger Pufferlösung gewaschen und gewünschtenfalls anschließend die Enzyme in wäßrigem Medium mittels bi- oder polyfunktioneller Verbindungen angekoppelt werden.

14. Verfahren zur Herstellung von permeabilisiertem und zusätzlich modifiziertem Schimmelpilzmycel nach einem der Ansprüche 9 bis 11, dadurch gekennzeichnet, daß die Ankopplung eines oder mehrerer zusätzlicher Enzyme an das Schimmelpilzmycel unter gleichzeitiger Zugabe von quaternärer Ammoniumverbindung erfolgt.

15. Verwendung von permeabilisiertem Schimmelpilzmycel nach einem der Ansprüche 1 bis 11 zur Durchführung biotechnischer Reaktionen.

16. Verwendung von permeabilisiertem Schimmelpilzmycel nach einem der Ansprüche 1 bis 8, als Ausgangsmaterial zur Gewinnung löslicher Glucoseoxidase und/oder Katalase.